# EUROPEAN PATENT APPLICATION

(11) **EP 1 391 726 A1**
(43) Date of publication of application: **25.02.2004**
(21) Application number: 01274270.6
(22) Date of filing: 10.12.2001
(51) Int. Cl.: G01N 33/00, H01F 1/00, G21K 5/00

(54) **APPARATUS AND METHOD FOR TRANSMITTING APPRECIABLE INFORMATION**

(30) Priority: 28.05.2001 JP 2001159565; 08.11.2001 JP 2001343057
(71) Applicant: Matsuoka, Sei, Shimabara-shi, Nagasaki 855-0059 (JP)
(72) Inventor: Matsuoka, Sei, Shimabara-shi, Nagasaki 855-0059 (JP)
(74) Representative: von Samson-Himmelstjerna, Friedrich R., Dipl.-Phys.
(86) International application number: PCT/JP2001/010811
(87) International publication number: WO 2002/097429

(57) **Abstract**

Energy generator means for transmitting quantitative value information from an object to another object is improved to efficiently transmit useful quantitative value information. A quantitative value information transmitting apparatus (1) comprises a group of rod magnets (10) including three rod magnets bundled with their polarities oriented in the same direction, a nonmagnetic cylindrical body (20) housing the group of rod magnets 10, a group of ionic electron emitting tubes (30) including 16 ionic electron emitting tubes helically arranged around the cylindrical body (20), and an ionic electron emitting body (40). A sending object (A) is placed at a forward position the transmitting apparatus (1) in the direction of energy transmission (D), and a receiving object (B) is placed at a further forward position. Magnetic wave energy containing quantitative value information is passed along the central axis surrounded by a converged magnetic flux from the group of rod magnets (10) and the cylindrical mirror plane of a (0) field produced by the group of ionic electron emitting tubes (30), and is emitted from the end of the transmitting apparatus (1), whereby the quantitative value information of the sending object (A) is transmitted to the receiving object (B).

## Description

### TECHNICAL FIELD

The invention relates to a technology for transmitting quantitative value information, and more particularly to an apparatus for transmitting quantitative value information which indicates the value of a biological relationship between an object and the pineal body part of a human brain, or a subject, and a method thereof.

### BACKGROUND ART

Every object (including substances) has a value intrinsic to the object, and it has heretofore been considered that the value is unique to the object itself. Recently, there are the cases, however, that when a subject grasps an object, an interrelationship between the subject and the object is grasped in terms of electromagnetic waves, and the interrelationship between the subject and the object is evaluated for a value, so that the information thereof (as employed herein, this information will be referred to as quantitative value information) is objectively evaluated even with consideration given to interest tolerance.

Among the known methods for evaluating such quantitative value information is one so-called O-ring test. The O-ring test is to determine whether or not an object is beneficial to a subject from the force by which a ring formed of a thumb and an index finger (this will be referred to as O-ring) is opened. Although not fully clarified scientifically yet, the O-ring test has been put to such uses as the treatment of intractable diseases in some medical institutions. US Patent No. 5188107 has proposed a concrete method for an O-ring test, and Unexamined Japanese Patent Application Publication No. Hei 10-67672 describes the use of an O-ring for the treatment of various intractable diseases.

The inventor has disclosed, in *Jintai Kagaku* [*Mind-Body Science*] (issued from the Society for Mind-Body Science) Vol. 8, No. 2, pp. 57-71, the experimental results that show the possibility of existence of a quantitative value information system composed of the pineal body part of a human brain and an O-ring. In *ibid*., Vol. 9, No. 2, pp. 41-56, the inventor has also verified the existence of the quantitative value information system and disclosed the results of experiments for transmitting and receiving this information by using an energy generator. The technique disclosed by these experimental results can be used to check and quantify a (0) and numerical value, and this technique differs from the so-called "O-ring test" in quality.

The following provides the concrete method of measuring a quantitative value of information described in *Jintai Kagaku* Vol. 8, No. 2. A red visible-light semiconductor laser device is placed on a desk. An ampule of 20% glucose is placed 20 cm ahead and is irradiated with the laser, and an assistant puts the index fingers of both of his/her hands through an O-ring of an examiner to pull open the O-ring with the right and left, a total of two fingers. Here, remaining closed is evaluated as (+), or (+1) at this stage. Next, in order to open the O-ring of this examiner, the second fingers of both the hands of the assistant for pulling apart laterally are added for greater interlocking force, and put through this O-ring to pull the O-ring open with the right and left, a total of four fingers. This stage corresponds to (+2), and if the O-ring is not opened yet, the assistant also puts the right and left third fingers through the O-ring of the examiner additionally to open with the right and left, a total of six fingers, which corresponds to (+3). The range of this closed (+) is still (+2), so that the object or the single ampule of 20% glucose is evaluated to have a value of (+2) which corresponds to the stage before the opening.

When an ampule of 5% glucose is placed 20 cm ahead of the laser device as an object and irradiated with the laser, the foregoing method results in (+5). Moreover, with an ampule of 50% glucose as the object, the foregoing method results in (-4). Furthermore, with 5 cc of hydrogen peroxide solution (H₂O₂) as the object, the foregoing method results in (-6).

Description will also be given of a method for measuring a quantitative value of information according to a physical equilibrium law across the laser device during laser emission. When the O-ring of a right hand is opened with laser irradiation, which means (-), this (-) is quantified by keeping pulling apart the O-ring of the right hand with a left finger touching the rear end of the laser device under emission until (-) is reached, and the resulting value is exactly the absolute value, or the (-)-signed quantitative value of information of the object which lies at a forward position of the laser device. Here, the polarities are symmetrical between the front and the rear, so that (+) at the rear end means (-) of the object in front while the absolute values are the same across the laser device.

When an ampule of 5% glucose (an intrinsic value of +5, from the foregoing result) and an ampule of 50% glucose (an intrinsic value of -4, from the foregoing result) are stacked at a forward position of this laser device, the quantitative value of information measures (+1). Similarly, an ampule of 5% glucose (an intrinsic value of +5) and an ampule of 20% glucose (an intrinsic value of +2) are stacked to measure (+7). Moreover, if an ampule of 5% glucose (an intrinsic value of +5) is placed at a forward position of the laser device, and an ampule of identical 5% glucose (an intrinsic value of +5) is put on the rear end of this laser device, the quantitative value of information measures (0).

From the foregoing experimental results, it is inferred that the quantitative values of information obtained by O-ring tests using the laser device have polarities and the addibility of quantified values. The use of this technique makes it possible to measure whatever high values.

In addition, the following proposal has been made in *Jintai Kagaku* Vol. 9, No. 2, pp. 41-56 as to an apparatus for transmitting (feeding in) the quantitative value information of an object to another object. That is, a red visible-light semiconductor laser device is used as a directional energy generator, a sending object for feeding quantitative value information into a receiving object is put beside a light inlet part of the laser device, and the quantitative value of information of the sending object is fed into the receiving object, a piece of white paper, at a distance of 10 cm or so.

In the experiment, (1) Minophagen C injection (antihepatitis, a quantitative value of information of (+20)), (2) 50 mg 1 V of TRH (thyrotropin-releasing factor)(a quantitative value of information of (+415)), and (3) Oncogen C-fos ab2 (a preparation embedded sample, oncogene, a quantitative value of information of (-16)) were used as sending objects for feeding quantitative value information into the receiving object. These three sending objects were put beside the light inlet parts of respective three laser devices in advance, and quantitative value information was fed into pieces of white paper, or receiving objects placed at a forward position of the respective laser devices. After 10 minutes, these three sending objects were put on the rear ends of the three laser devices, respectively, and the pieces of white paper were irradiated with the laser devices. The measured quantitative value of information was (0). It was confirmed that the pieces of white paper had the same values of quantitative value information as the quantitative values of information of the sending objects fed in, or that the quantitative value information of the sending objects was projected and left in the pieces of white paper, the receiving objects. The same results were also seen after a lapse of 24 hours and a lapse of one week, with no variation (attenuation) of the quantitative values of information within these periods. These three are free from confusion, having a one-to-one correspondence.

Based on the foregoing verifications, in order to transmit more complicated macromolecular biological object as the quantitative value of information system, the inventor has also proposed a transmitting apparatus which achieves three-dimensional higher integration of the quantitative value of information. This apparatus comprises two red visible-light semiconductor laser devices (A, B) having side inputs for accepting different pieces of information, and a laser demodulator (light receiving and transferring apparatus). The laser demodulator converts an electric signal input into an optical signal, which converts an optical signal into an electric signal, and then returns the electric signal into an optical signal.

The above-described apparatus was used with (1) Brain Derived Neurotrophic Factor: 5-µg RBL (brain derived neurotrophic factor, a quantitative value of information of (+489)), (2) human β Nerve Growth Factor: 10-µg PTEC (human nerve growth factor, a quantitative value of information of (+489)), and (3) bcl2 Trevigen: 100-pmol/ut (oncogene, apoptosis inhibition, a quantitative value of information of (+489)) as sending objects. The three were bundled with a rubber band and placed on the near side of the side input of the laser device (A), and a piece of white paper, or the receiving object, 10 cm at a forward position of the laser device (B) was irradiated through the laser demodulator for 20 seconds so that the quantitative value information of the three was fed in. A narrow portion of the light spot fed onto this white paper was measured for a quantitative value of information, and the quantitative value of information measured (+1854). The three sending objects bundled themselves had a quantitative value of information of (+810) in total, whereas it is inferred that the original value of the quantitative value information (+810) was integrated and increased to (+1854) by passing through the foregoing transmitting apparatus.

As described above, the inventor has invented the technology of transmitting the information for making objective evaluation of the mutual relationship between a subject and an object to be measured by the proper "O-ring test" even with values and interests taken into account, i.e., the quantitative value of information from an object to another object, and has proposed a laser-based energy generator as the directional energy generator which constitutes the transmitting apparatus. As described above, when this energy generator is used as the energy generator of a quantitative value information transmitting apparatus, it is possible to transmit quantitative value information with its original quantitative value of information integrated and amplified. The degree of integration remains on the order of several hundreds to a thousand times or above, however, and it has been found that higher integration is difficult to achieve by using laser-based energy generators.

A problem to be solved by the present invention is to improve the energy generating means for transmitting quantitative value information from an object to another object, and make it possible to transmit beneficial quantitative value information with higher efficiency.

### DISCLOSURE OF THE INVENTION

As a result of intensive studies in order to solve the foregoing problem, the inventor has conceived to use a magnetism generator and an ion generator in combination instead of the laser device as the directional energy generator for use in the apparatus for transmitting the quantitative value of information, and thus completed the present invention.

That is, the present invention is a quantitative value information transmitting apparatus for transmitting quantitative value information of a sending object to a receiving object by using a directional energy generator, in which the energy generator is made of a combination of a magnetism generator and an ion generator.

According to the energy generator of the present invention, the magnetism generator and the ion generator produce a synergistic effect of providing a quantitative value of information integrated several thousand to several tens of thousands times or more as compared to those of quantitative value information transmitting apparatuses using conventional laser devices. The quantitative value of information that is integrated into a high value and transmitted by this energy generator is then stored in the receiving spot for or beyond a predetermined period stably without attenuation.

Here, the magnetism generator may be composed of a plurality of rod magnets bundled with their polarities oriented in the same direction, and the ion generator may have the configuration that an even number of ionic electron emitting tubes surrounding the rod magnets are arranged so that adjoining ones of the ionic electron emitting tubes are opposite to each other in polarity.

When the magnetism generator is composed of the plurality of rod magnets bundled with their polarities oriented in the same direction, the magnetic wave energy occurring from the magnets becomes directional, and the mutual repulsion between the same polarities enhances the magnetic wave energy.

Moreover, when the ion generator is composed of the even number of ionic electron emitting tubes surrounding the rod magnets and adjoining ones of the ionic electron emitting tubes are arranged with their polarities opposite to each other, (+) and (-) of the ionic electron emitting tubes cancel each other to form a cylindrical field of ionic (0). This cylindrical (0) field makes a mirror plane for reflecting magnetic waves, and the magnetic wave energy passing through this cylindrical field is reflected by the mirror plane to cause mutual interference so that the magnetic wave energy is converged to the straightforward direction. While magnetic wave energy containing quantitative value information proceeds through this cylindrical field, the magnetic wave energy is integrated and enhanced to integrate the quantitative value information. Incidentally, the ionic electron emitting tubes may use ones that are conventionally used as light therapy apparatuses with their ion emitting ports in contact with painful or such spots.

To be more specific, it is desirable to provide the configuration that the plurality of rod magnets are housed in a nonmagnetic cylindrical body, such as a cylindrical body made of glass or plastic, and this cylindrical body is surrounded by 10 to 16 of ionic electron emitting tubes. When the rod magnets are housed in the nonmagnetic cylindrical body, the magnetism generator (rod magnets) and the ion generator (ionic electron emitting tubes) are electromagnetically isolated from each other. This prevents the magnetic waves and the ion flow from being mixed and disturbed, so that the magnetic waves travel in a constant direction to converge while repeating reflection and interference through the cylindrical (0) ion flow, and quantitative value information is integrated and enhanced with precision.

Furthermore, it is desirable to provide the configuration that the ionic electron emitting tubes surrounding the cylindrical body are arranged with their extremities helically shifted back clockwise in the direction of emission. Consequently, (+) ions and (-) ions emitted adjacently cancel each other into ionically (0), and the ion outputs of (0) value lie around helically clockwise to form a (0) mirror plane made of (0) values. Here, the (0) mirror plane made of (0) values refers to the field of (0) values formed by the ionic electron emitting tubes surrounding the cylindrical body housing the rod magnets, and magnetic wave energy containing quantitative value information passes along the central axis of the cylinder of the mirror plane encircled by the (0) value planes. This magnetic wave energy travels straight while repeating reflection and interference on the cylindrical mirror plane for convergence, whereby the quantitative value information is integrated further. It is inferred that the magnetic waves travel straight as enhanced and stabilized by the reflection on the helical mirror plane of (0) values, bearing a great resemblance to the transformation of light passing through a mirror-lined box into laser. The technology of integrating such significant factors as p53 and NGF mentioned above and fixing the same in a certain position seems to have effective uses.

It is also desirable that the S poles, or the poles of the rod magnets constituting the magnetism generator, be arranged toward the end in the direction of energy transmission. When the S poles of the rod magnets are directed toward the end, the S magnetic field activates biological objects and the N magnetic field inactivates biological objects. Thus, directing the S magnetic field toward the end functions to increase the capability of integrating quantitative value information.

The quantitative value information transmitting method of the present invention which uses the quantitative value information transmitting apparatus described above is a method comprising the steps of: placing a sending object at a forward position of the energy generator made of the combination of the magnetism generator and the ion generator; placing a receiving object at a further forward position of this sending object; and transmitting quantitative value information of the sending object to the receiving object by using directional energy of the energy generator (the magnetism generator and the ion generator).

The quantitative value information transmitted by this method, as is evident from experimental examples to be described later, has transmission capability far higher than in the case of transmission using a conventional laser device. Capability of penetration even through a reinforced concrete structure has been confirmed.

### BRIEF DESCRIPTION OF THE DRAWINGS

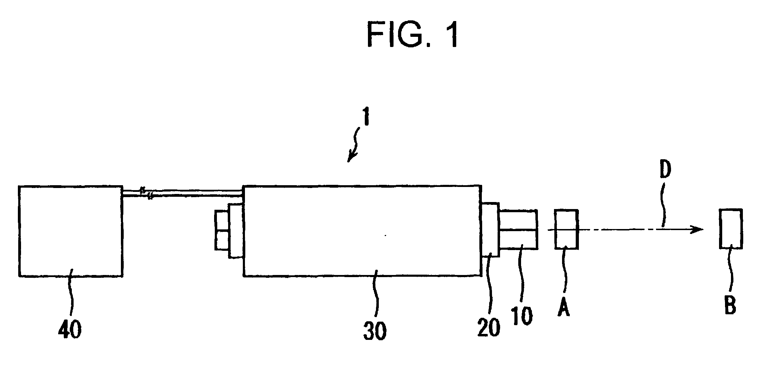

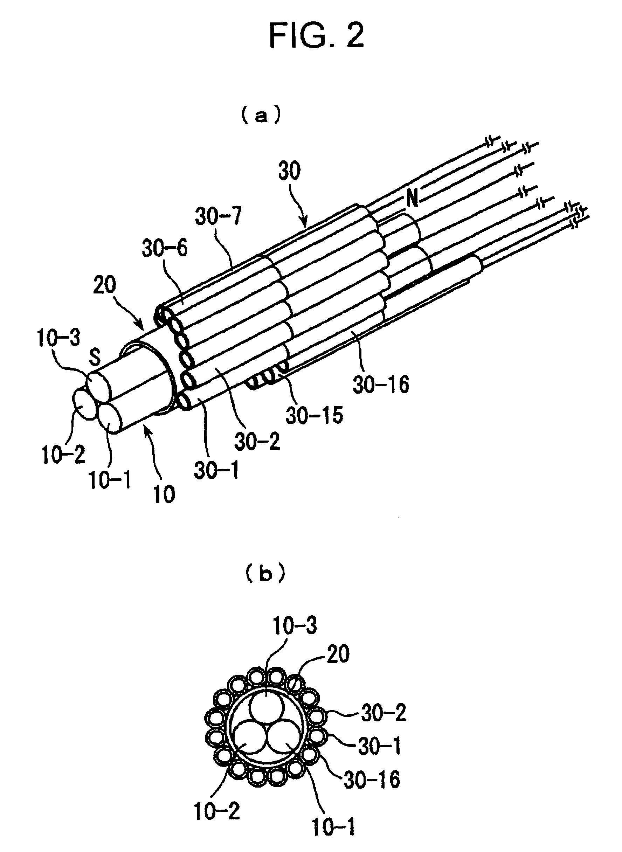

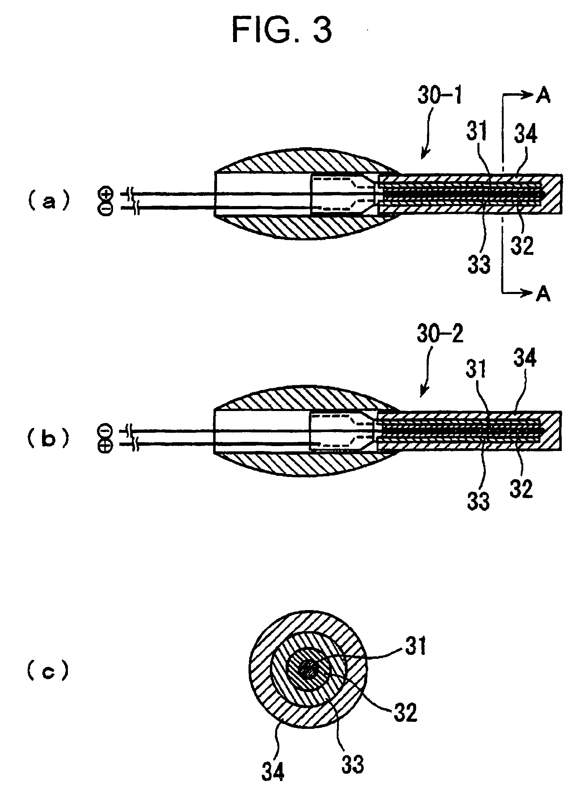

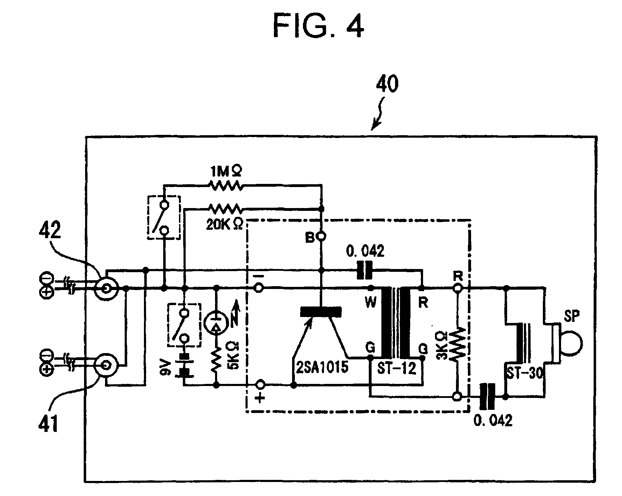

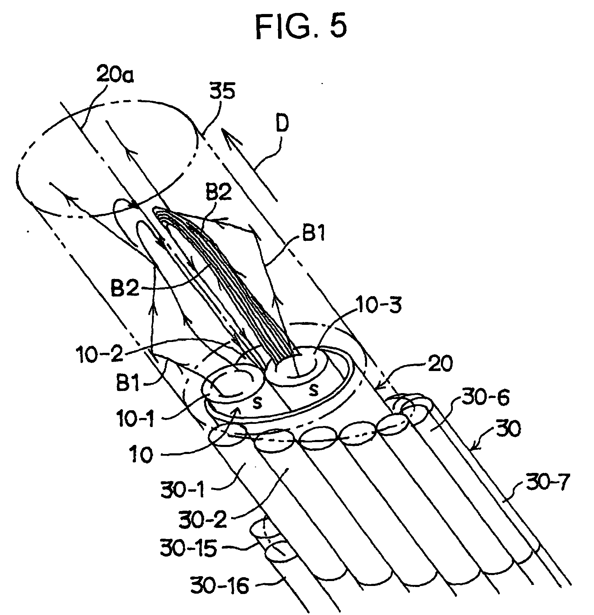

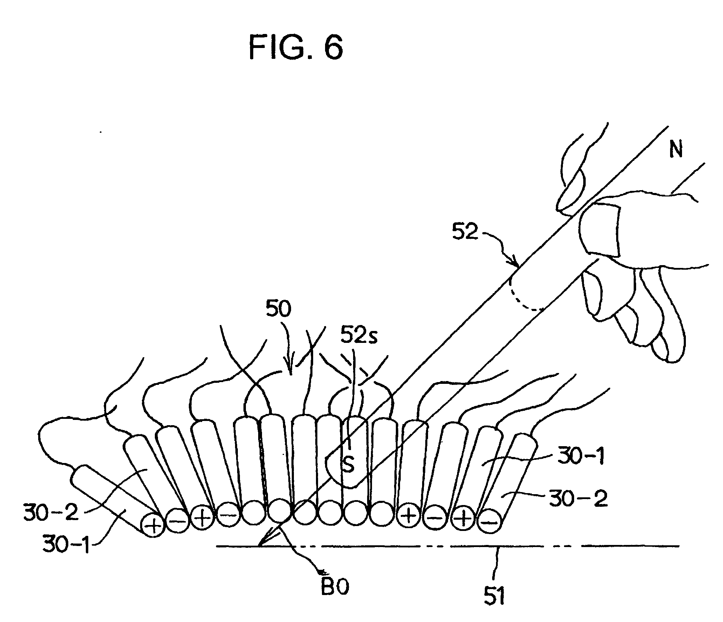

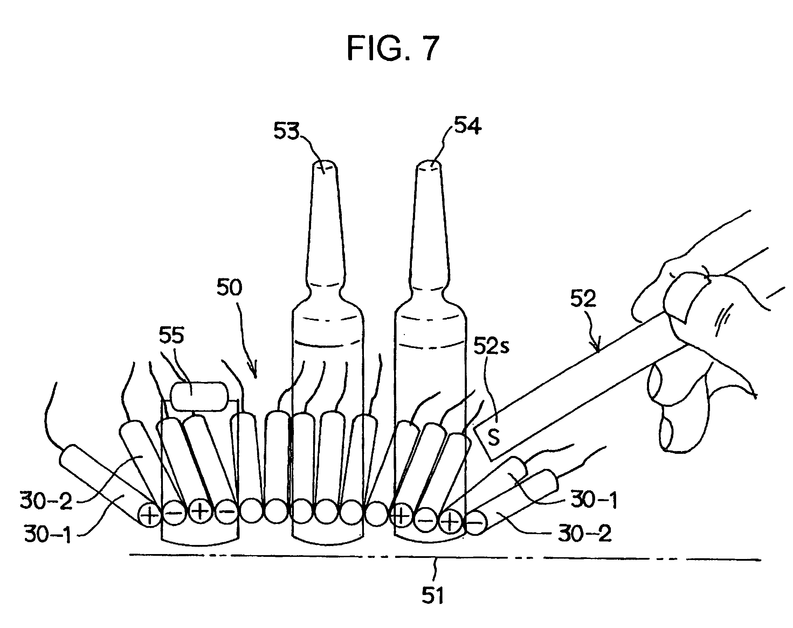

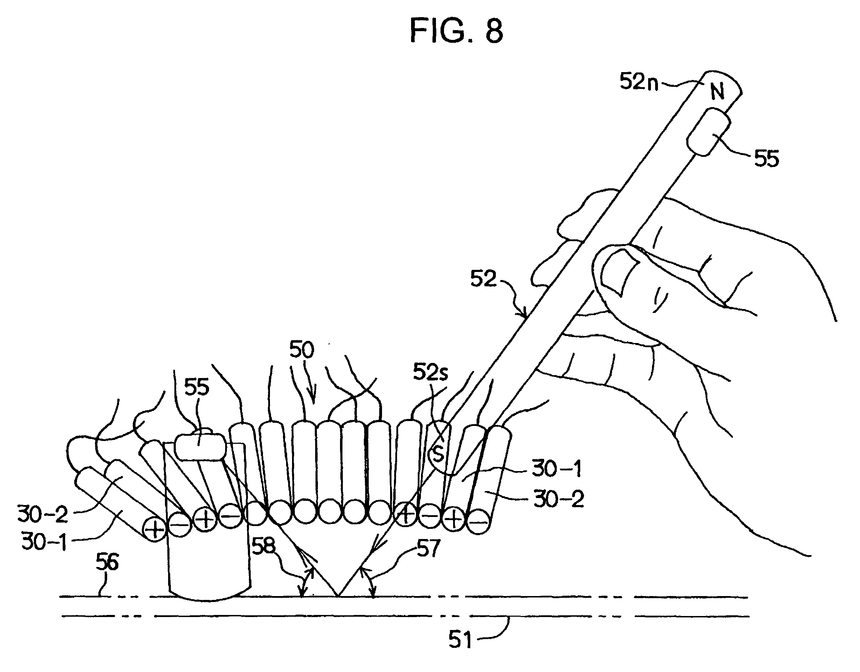

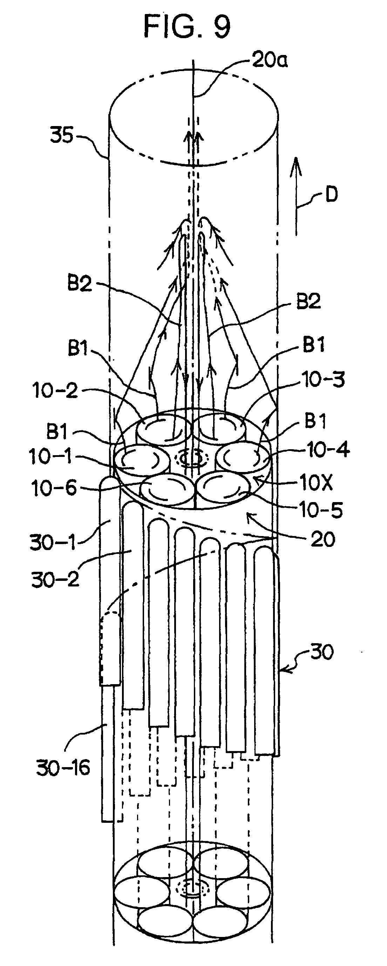

Fig. 1 is a diagram showing the basic configuration of a quantitative value information transmitting apparatus according to an embodiment of the present invention. Fig. 2 is a diagram showing the combined structure of a group of rod magnets, a nonmagnetic cylindrical body, and a group of ionic electron emitting tubes, (a) being a perspective view and (b) a side view taken from the end side. Fig. 3 is a diagram showing the sectional structures of the ionic electron emitting tubes and the connection of different polarities, (a) showing the connection of positive polarity, (b) the connection of negative polarity, and (c) a sectional view taken along the line A-A. Fig. 4 is a diagram showing the electric circuit of an ionic electron emitter body. Fig. 5 is a partial perspective view showing the combined structure of a group of rod magnets, a nonmagnetic cylindrical body, and a group of ionic electron emitting tubes. Fig. 6 is a partial perspective view showing the state of an experiment using a row of ionic electron emitting tubes and a rod magnet. Fig. 7 is a partial perspective view showing the state of an experiment using the row of ionic electron emitting tubes and the rod magnet. Fig. 8 is a partial perspective view showing the state of an experiment using the row of ionic electron emitting tubes and the rod magnet. Fig. 9 is a partial perspective view showing another embodiment as to the combined structure of a group of rod magnets, a nonmagnetic cylindrical body, and a group of ionic electron emitting tubes.

### BEST MODE FOR CARRYING OUT THE INVENTION

Fig. 1 is a diagram showing the basic configuration of a quantitative value information transmitting apparatus according to an embodiment of the present invention. A quantitative value information transmitting apparatus 1 of the present embodiment comprises a group of rod magnets 10 including three rod magnets bundled with their polarities oriented in the same direction, a nonmagnetic cylindrical body 20 housing the group of rod magnets 10, a group of ionic electron emitting tubes 30 including 16 ionic electron emitting tubes helically arranged around the cylindrical body 20, and an ionic electron emitter body 40.

A sending object A is placed at a forward position of this transmitting apparatus 1 in the direction of energy transmission D, and a receiving object B is placed at a further forward position. Magnetic wave energy containing quantitative value information is passed along the central axis surrounded by a converged magnetic flux from the group of rod magnets 10 and the cylindrical mirror plane of a (0) field produced by the group of ionic electron emitting tubes 30, and is emitted from the end of the transmitting apparatus 1, whereby the quantitative value information of the sending object A is transmitted to the receiving object B.

Fig. 2 is a diagram showing the combined structure of the group of rod magnets, the nonmagnetic cylindrical body, and the group of ionic electron emitting tube, in which (a) is a perspective view, and (b) a side view taken from the end side.

The group of rod magnets 10 is a bundle of three rod magnets 10-1 to 10-3 having their S poles on the end side (on the left in Fig. 2(a)). This group of rod magnets 10 is housed in the cylindrical body 20 which is made of nonmagnetic material (glass or plastic). The rod magnets 10-1 to 10-3 are 320-gauss rod magnets of column shape, having an outer diameter of 10 mm and a length of 153 mm. Sixteen ionic electron emitting tubes 30-1 to 30-16 are helically disposed around the cylindrical body 20. The ionic electron emitting tubes 30-1 to 30-16 are arranged with their respective extremities helically shifted back clockwise in the direction of emission (to the left in Fig. 2(a)), and are connected so that adjoining ones of the ionic electron emitting tubes are opposite to each other in polarity.

Fig. 3 is a diagram showing the sectional structures of the ionic electron emitting tubes and the connections of different polarities, in which (a) shows the connection of positive polarity, (b) shows the connection of negative polarity, and (c) is a sectional view taken along the line A-A.

The ionic electron emitting tubes 30-1 (odd-numbered) and 30-2 (even-numbered) have the same structure except for the wire connection. The essential parts or the extremities comprise, in order from the center: a center electrode 31 which is made of metal (plated brass, in the present embodiment) for causing discharge to emit ionized positrons or electrons; a vinyl chloride tube 32 which is an insulator; a pipe 33 which is made of metal (plated brass, in the present embodiment) for accumulating electrons of opposite type from that of the center electrode 31; and a vinyl chloride tube 34 (the tube having an inner diameter of 5 mm and an outer diameter of 6.5 mm) which is an insulator. When the group of ionic electron emitting tubes 30 having such a structure is energized with the ionic electron emitter body 40, (+) ions and (-) ions are emitted in the direction D in Fig. 1.

This group of ionic electron emitting tubes 30 itself and the energy generator itself having the group of rod magnets 10 combined were measured for respective pieces of quantitative value information. It was confirmed from the experiment that the greater the number of ionic electron emitting tubes was, the greater the quantitative value of information of the group of ionic electron emitting tubes 30 itself becomes, and that the quantitative value of information becomes even higher when an even number of ionic electron emitting tubes were arranged as helically shifted back one by one from the right. Moreover, the combination with the group of rod magnets 10 realizes linear convergence of magnetic waves and increases the quantitative value of information of the energy generator further. The energy generator itself according to the present embodiment actually measured (+9712) in the quantitative value of information.

Fig. 4 is a diagram showing the electric circuit of the ionic electron emitter body. The ionic electron emitter body 40 is basically the same circuit as the electric circuit of an electron ion beam apparatus that is used as a light therapy apparatus with its ion radiation port in contact with a painful or such spot. A terminal 41 of this ionic electron emitter body 40 is connected with the odd-numbered ionic electron emitting tubes out of the group of ionic electron emitting tubes 30, and a terminal 42 is connected with the even-numbered ionic electron emitting tubes out of the group of ionic electron emitting tubes 30, thereby forming the quantitative value information transmitting apparatus 1 shown in Fig. 1.

Now, with reference to Fig. 5, description will be given of a cylindrical mirror plane 35 and magnetic lines of force B1, B2 which occur in front of the group of rod magnets 10 and the group of ionic electron emitting tubes 30.

In the quantitative value information transmitting apparatus 1 having the configuration described previously, when the ionic electron emitter body 40 is activated to energize the group of ionic electron emitting tubes 30, as shown in Fig. 5, the ion emitting tubes 30-1 to 30-16 emit respective (+) ions and (-) ions in the direction D. These (+) ions and (-) ions cancel each other to reach (0) ionically, and the ion outputs of (0) values lie around helically clockwise to form the cylindrical mirror plane 35 made of the (0) values.

Meanwhile, the magnetic lines of force B1 released from the edge portions (portions closer to the cylindrical body 20) of the group of three rod magnets 10 bundled travel in the direction D while repeating reflection on the cylindrical mirror plane 35. The magnetic lines of force B2 released from mutual opposing portions (portions closer to a central axis 20a of the cylindrical body 20) of the group of rod magnets 10 travel in the direction D with mutual repulsion, reach a critical distance to turn around, and return to the N pole of the group of rod magnets 10 along the central axis 20a of the cylindrical body 20 while approaching each other to the utmost limit. That is, these magnetic lines of force B1 and B2 form magnetic wave energy, and this magnetic wave energy is reinforced by the mutual repulsion of the same polarities.

As above, magnetic wave energy containing quantitative value information passes through the cylindrical mirror plane 35 of ionic (0) values, and this magnetic wave energy repeats reflection and interference for convergence due to the cylindrical mirror plane 35, whereby the quantitative value information is integrated further.

Now, with reference to Figs. 6 to 8, verification will be conducted on the relationship between the field of ionic (0) values and the magnetic lines of force which are formed at a forward position of the group of ion emitting tubes 30.

As shown in Fig. 6, the ion emitting tubes 30-1 for emitting (+) ions and the ion emitting tubes 30-2 for emitting (-) ions were arranged alternately to form a row of ion emitting tubes 50. These ion emitting tubes 30-1 and 30-2 were energized to the polarities shown in Figs. 3(a) and 3(b), and a rod magnet 52 was directed aslant with its S pole 52s toward an imaginary plane 51 in front of the row of ion emitting tubes 50, in which state the quantitative value information measured a (0) value. It can be said that this shows that the imaginary plane 51 forms a mirror plane of ionic (0) value and the magnetic lines of force are reflected. In other words, it is inferred that when a magnetic line of force B0 occurring from the S pole 52s of the rod magnet 52 was incident, the quantitative value information became (0) due to the reception of the (0) information of this imaginary plane 51.

Next, as shown in Fig. 7, an ampule of 5% glucose 53 (a quantitative value of information of +5), an ampule of 50% glucose 54 (a quantitative value of information of -4), and a capsule of EPA. DHA 55 (a quantitative value of information of +80) are placed as objects on the imaginary plane 51 that is formed as in Fig. 6 described above. The S pole 52s of the aslant rod magnet 52 is directed toward the respective objects, in which case all the quantitative values of information become (0). It is inferred that the quantitative value information intrinsic to the respective objects becomes (0) in value by receiving the information on the imaginary plane 51 which has an ionically (0) value.

Next, as shown in Fig. 8, the single capsule of EPA. DHA 55 (a quantitative value of information of +80) was placed on an imaginary plane 56 above the imaginary plane 51 that was formed as in Fig. 6 described above, and the S pole 52s of the aslant rod magnet 52 was directed toward the imaginary plane 56 with the single capsule of EPA. DHA 55 (a quantitative value of information of +80) attached to an N pole 52n thereof. A position of (0) was determined and found to fall on the position where the magnetic line of force occurring from the S pole 52s was reflected by the imaginary plane 56 with the same angles of incidence 57 and reflection 58. This shows that the single capsule of EPA. DHA 55 on the imaginary plane 56 and the single capsule of EPA. DHA 55 on the N pole 52n cancel into (0) since the magnetic force of line and the line reflected from the (0) plane are physically at the same angle and the S pole 52s of the rod magnet 52 is (+) in front and (-) behind. Incidentally, if the rod magnet 52 is directed perpendicular to the imaginary plane 51 with the S pole 52s downward, it is expected that the magnetic line of force will pass through the imaginary plane 51 with no reflection.

From the foregoing, it is found that the relationship between the ionically (0) imaginary plane 51 and the magnetic line of force of the rod magnet 52 is almost equivalent to the relationship between a mirror and a beam.

Next, with reference to Fig. 9, description will be given of another embodiment as to the combined structure of the group of rod magnets 10, the nonmagnetic cylindrical body 20, and the group of ionic electron emitting tubes 30 shown in Figs. 2 and 5. In Fig. 9, members that have the same structures and functions and exhibit the same effects as those of the members shown in Figs. 2 and 5 will be designated by the same reference numerals as in Figs. 2 and 5, and the description thereof will be omitted.

As shown in Fig. 9, according to the present embodiment, a group of rod magnets 10X made of six rod magnets 10-1 to 10-6 bundled with their S poles in the direction of energy transmission D (to the left in Fig. 9) is housed inside a nonmagnetic cylindrical body 20. Twenty to twenty-two ionic electron emitting tubes (30-1 up to 30-16 are shown in Fig. 9) are helically disposed around the cylindrical body 20.

As above, the group of rod magnets 10x formed by bundling the six rod magnets 10-1 to 10-6 provided double the number of rod magnets as compared to the case where the group of rod magnets 10 formed by bundling the three rod magnets 10-1 to 10-3 is disposed. What is more, the number of ionic electron emitting tubes is increased from 16 to 20-22. The magnetic energy for transmitting quantitative value information is thus enhanced further, and the field of ionically (0) values is extended and enhanced in mirror area. This means a further increase of the quantitative value of information in experiments. Consequently, superior functions and effects can be obtained in terms of quantitative value information transmission.

Hereinafter, description will be given of experimental examples in which the transmitting apparatus 1 of the present invention is used.

### [Experimental Example 1]

The end of the transmitting apparatus 1 was put into contact with a 70-cm-height position of the first reinforced concrete post out of seven reinforced concrete posts (approximately 70 cm in thickness) erected on a line in a section of approximately 35 m, and energy was radiated toward the seventh concrete post. After energy radiation of approximately 10 seconds, the seventh concrete post was measured for a quantitative value of information. A circular range of approximately 4 cm in diameter on the back of the post, at a position of approximately 70 cm from the floor, measured (+9712) in the quantitative value of information. As described above, since the energy generator itself of the present embodiment has a quantitative value of information of (+9712), it can be seen that the quantitative value of information of this energy generator was transmitted to the seventh concrete post even through the concrete without attenuation.

### [Experimental Example 2]

An ampule of 5% glucose was placed at a position 50 m ahead of the transmitting apparatus 1, and energy was radiated from the transmitting apparatus 1 toward the single ampule of 5% glucose in front with an ampule of 5% glucose put on the rear end of the transmitting apparatus 1 (the N-pole side of the group of rod magnets 10). In this state, an O-ring test was conducted, which showed a quantitative value of information of (0). This seems to show that the objects in front and behind resonate into (0) when they are equivalent in terms of electromagnetics, and shows that the transmitting apparatus 1 transmits quantitative value information with extremely high precision in view of the figures of the quantitative values of information and the polarity addibility described above. Under the same condition, an ampule of physiological saline was tested, and the result was the same.

### [Experimental Example 3]

A 2-cc ampule of 0.5% Xylocaine injection (a quantitative value of information of (+2)) as a sending object was put on the end of the transmitting apparatus 1 (the S-pole side of the group of rod magnets 10), and energy was radiated for 10 seconds from the transmitting apparatus 1 toward iron powder dispersed and precipitated on the bottom of an oil tank through the side thereof. In a period from the day after the energy radiation to 50 days later, O-ring tests using the transmitting apparatus 1 having a 2-cc ampule of 0.5% Xylocaine injection put on the rear end thereof (the N-pole side of the group of rod magnets 10) were conducted to determine if the quantitative value information transmitted to the receiving object, iron powder, remained. As a result, it was confirmed that the transmitted quantitative value information remained in the iron powder lying in the range of energy transmission from the transmitting apparatus 1 even after 50 days. Under the same condition, a test was conducted using p53 (cancer inhibitor) hum. 250pM. BIOGNOSTIK (a quantitative value of information of (+9887)) as the sending object, which showed the same result that the quantitative value information of p53 transmitted to the iron powder in the range of energy transmission from the transmitting apparatus 1 remained. More specifically, (0) resulted from an O-ring test that was conducted with the S poles of the rod magnets toward the iron powder in the oil tank and with p53 on the N poles of the rod magnets. This shows that the objects in front and behind are equivalent.

### [Experimental Example 4]

When energy was radiated from the transmitting apparatus 1 to an egg (a quantitative value of information of (+6)) for 10 seconds, the quantitative value information of the transmitting apparatus 1 itself was transmitted to the egg. Moreover, when p53 of the experimental example 3 was put as a sending object on the end of the transmitting apparatus 1, the quantitative value information of p53 was transmitted to the egg additionally. The quantitative value of information of the egg resulted in (+19605), that is the sum total of the original quantitative value of information of the egg (+6), the quantitative value of information of the transmitting apparatus 1 itself (+9712), and the quantitative value of information of p3 (+9887).

### [Experimental Example 5]

When NGF (human β Nerve Growth Factor: 10 µg PTEC, nerve growth factor, a quantitative value information of (+811)) was put as a sending object on the end of the transmitting apparatus 1, and energy was radiated from the transmitting apparatus 1 to commercially available packed milk (a quantitative value of information of (+6)), the milk resulted in having a quantitative value of information of (+10529). This quantitative value of information was also confirmed by the same method even after 50 days of refrigeration. The value is the sum total of the original quantitative value of information of milk (+6), the quantitative value of information of the transmitting apparatus 1 itself (+9712), and the quantitative value of information of NGF (+811), showing that this value remains unchanged even after 50 days of refrigeration.

### [Experimental Example 6]

When energy was radiated from the transmitting apparatus 1 to commercially available mineral water (a quantitative value of information of (+6)), the quantitative value of information of the transmitting apparatus 1 itself (+9712) was added to the original quantitative value of information of the mineral water (+6) so that the mineral water resulted in having a quantitative value of information of (+9718).

### INDUSTRIAL APPLICABILITY

A new quantitative value information system governed by the pineal body part of a human brain and an O-ring has been found, and a new index for learning efficacy has becoming clear from the principle and application thereof. This information system has of great importance as means for evaluating the quantitative interrelationship between a human being and an object in numerical terms objectively. For example, the present invention may be applied to confirmation of efficacy of medicines where the efficacy is clarified by measuring it in objective numerical terms in view of the relationship between the medicines and the person who takes the medicines, and also to determination of the effectiveness and affinity of food and drink to human bodies when developing new food and drink. It is also applicable to transmit and fix effective application information to target objects.

## Claims

1. A quantitative value information transmitting apparatus for transmitting quantitative value information of a sending object to a receiving object by using a directional energy generator, wherein said energy generator is made of a combination of a magnetism generator and an ion generator.

2. The quantitative value information transmitting apparatus according to claim 1, wherein said magnetism generator is composed of a plurality of rod magnets bundled with their polarities oriented in the same direction; said ion generator is composed of an even number of ionic electron emitting tubes surrounding said rod magnets; and adjoining ones of said ionic electron emitting tubes are arranged with their polarities opposite to each other.

3. The quantitative value information transmitting apparatus according to claim 2, wherein said plurality of rod magnets are housed in a nonmagnetic cylindrical body; and said cylindrical body is surrounded by 10 to 16, an even number of ionic electron emitting tubes.

4. The quantitative value information transmitting apparatus according to claim 3, wherein said ionic electron emitting tubes surrounding said cylindrical body housing said rod magnets are arranged with their extremities helically shifted back clockwise in the direction of emission.

5. In a method of transmitting quantitative value information using the quantitative value information transmitting apparatus according to any one of claims 1 to 3 having a magnetism generator and an ion generator as a directional energy generator, the method comprising the steps of: placing a sending object at a forward position of said energy generator; placing a receiving object at a further forward position of said sending object; and transmitting quantitative value information of said sending object to said receiving object by using directional energy of said energy generator.

## Amended claims

### Amended claims under Art. 19.1 PCT

**1.** (Amended) A quantitative value information transmitting apparatus for transmitting quantitative value information of a sending object to a receiving object by using a directional energy generator, wherein said energy generator is made of a magnetism generator composed of a plurality of rod magnets bundled with their polarities oriented in the same direction and an ion generator composed of an even number of ionic electron emitting tubes surrounding said plurality of rod magnets, adjoining ones of said ionic electron emitting tubes being arranged with their polarities opposite to each other.

**2.** (Canceled)

**3.** (Amended) The quantitative value information transmitting apparatus according to claim 1, wherein said plurality of rod magnets are housed in a nonmagnetic cylindrical body; and said nonmagnetic cylindrical body is surrounded by 10 to 16, an even number of ionic electron emitting tubes.

**4.** The quantitative value information transmitting apparatus according to claim 3, wherein said ionic electron emitting tubes surrounding said cylindrical body housing said rod magnets are arranged with their extremities helically shifted back clockwise in the direction of emission.

**5.** (Amended) In a method of transmitting quantitative value information by using the quantitative value information transmitting apparatus according to any one of claims 1, 3, and 4 having a magnetism generator and an ion generator as a directional energy generator, the method comprising the steps of: placing a sending object at a forward position of said energy generator; placing a receiving object at a further forward position of said sending object; and transmitting quantitative value information of said sending object to said receiving object by using directional energy of said energy generator.
